# EUROPEAN PATENT APPLICATION

(11) **EP 4 226 790 A1**
(43) Date of publication of application: **16.08.2023**
(21) Application number: 21931523.1
(22) Date of filing: 17.03.2021
(51) Int. Cl.: A24F 40/465, A24F 40/57

(54) **SUCTION DEVICE, PROGRAM, AND SYSTEM**

(71) Applicant: Japan Tobacco Inc., Tokyo 105-6927 (JP)
(72) Inventor: SERITA, Kazutoshi, Tokyo 130-8603 (JP); KAWASAKI, Reijiro, Tokyo 130-8603 (JP)
(74) Representative: Hoffmann Eitle
(86) International application number: PCT/JP2021/010851
(87) International publication number: WO 2022/195771

(57) **Abstract**

[Problem] To provide a structure that enables preferential generation of aerosol in an induction heating-type suction device. [Solution] This suction device is provided with: a power supply unit for supplying DC power; a drive circuit including an inverter circuit for converting the DC power supplied from the power supply unit to AC power and an electromagnetic induction source for generating a variable magnetic field using the AC power supplied from the inverter circuit; a control unit for controlling the supply of power from the power supply unit to the drive circuit; a holding unit for holding a substrate containing an aerosol source; and a power sensor for detecting information of the DC power supplied from the power supply unit to the drive circuit. The control unit controls the supply of power to the drive circuit on the basis of information relating to a disturbance and information on the DC power supplied to the drive circuit.

## Description

### Technical Field

The present invention relates to an inhaler device, a program, and a system.

### Background Art

Inhaler devices, such as e-cigarettes and nebulizers, for generating a substance to be inhaled by users are widespread. For example, the inhaler devices generate an aerosol having a flavor component imparted thereto, by using a substrate including an aerosol source for generating the aerosol, a flavor source for imparting the flavor component to the generated aerosol, and the like. Users can enjoy the flavor by inhaling the aerosol having the flavor component imparted thereto, which is generated by the inhaler devices. An action of a user inhaling an aerosol is hereinafter referred to as a puff or a puff action.

Inhaler devices using an external heat source such as a heating blade had been dominant until recently. In recent years, however, inhaler devices of induction heating type have been attracting attention. For example, Patent Literature 1 below discloses a technique of estimating a temperature of a susceptor included in a substrate from an apparent electrical resistance value when the susceptor is heated by induction heating.

### Citation List

### Patent Literature

Patent Literature 1: JP 6623175 B2

### Summary of Invention

### Technical Problem

Inhaler devices using an external heat source measure and control a temperature of the external heat source to implement appropriate generation of an aerosol. In contrast, inhaler devices of induction heating type have difficulty in directly measuring and controlling the temperature of the susceptor and thus in implementing appropriate generation of an aerosol. As disclosed in Patent Literature 1 above or the like, the technique of estimating a temperature of a susceptor has been developed. However, there is room for improvement in the accuracy of such a technique.

Accordingly, the present invention has been made in view of the issue described above, and it is an object of the present invention to provide a mechanism that enables an inhaler device of induction heating type to appropriately generate an aerosol.

### Solution to Problem

To overcome the issue described above, an aspect of the present invention provides an inhaler device including: a power supply configured to supply DC electric power; a drive circuit including an inverter circuit configured to convert the DC electric power supplied from the power supply into AC electric power, and an electromagnetic induction source configured to generate a varying magnetic field by using the AC electric power supplied from the inverter circuit; a controller configured to control electric power supply from the power supply to the drive circuit; a holder configured to hold a substrate including an aerosol source; and an electric power sensor configured to detect information of the DC electric power supplied from the power supply to the drive circuit, in which the electromagnetic induction source is disposed at a position where the varying magnetic field generated from the electromagnetic induction source penetrates a susceptor that is disposed in thermal proximity to the aerosol source included in the substrate held by the holder, the susceptor is configured to produce heat upon being penetrated by the varying magnetic field, and the controller is configured to control the electric power supply to the drive circuit, based on information related to a disturbance and the information of the DC electric power supplied to the drive circuit detected by the electric power sensor.

The controller may be configured to estimate a temperature of the susceptor, based on the information related to the disturbance and the information of the DC electric power supplied to the drive circuit, and control the electric power supply to the drive circuit, based on the estimated temperature of the susceptor.

The controller may be configured to correct, based on the information related to the disturbance, the temperature of the susceptor estimated based on the information of the DC electric power supplied to the drive circuit.

The inhaler device may further include a first temperature sensor configured to detect a temperature of an external environment, in which the information related to the disturbance may include the temperature of the external environment.

The controller may be configured to correct the temperature of the susceptor estimated based on the information of the DC electric power supplied to the drive circuit, to be lower as the temperature of the external environment is higher and to be higher as the temperature of the external environment is lower.

The information related to the disturbance may include an electrical resistance value of the drive circuit.

The controller may be configured to correct the temperature of the susceptor estimated based on the information of the DC electric power supplied to the drive circuit, to be lower as the electrical resistance value of the drive circuit is larger and to be higher as the electrical resistance value of the drive circuit is smaller.

The inhaler device may further include a second temperature sensor configured to detect a temperature of the drive circuit, in which the controller may be configured to estimate the electrical resistance value of the drive circuit, based on the temperature of the drive circuit detected by the second temperature sensor.

The controller may be configured to estimate the electrical resistance value of the drive circuit, based on an elapsed time from a start of the electric power supply to the drive circuit and information that is stored in advance and indicates a time-series change in the electrical resistance value of the drive circuit.

The controller may be configured to control a voltage to be applied to the drive circuit to control the electric power supply to the drive circuit.

The controller may be configured to control, based on a heating profile, the electric power supply to the drive circuit, the heating profile being information that defines a time-series change in a target temperature that is a target value of a temperature of the susceptor.

The controller may be configured to perform control to apply a first voltage to the drive circuit in a case where an estimated temperature of the susceptor is lower than the target temperature, and to apply a second voltage to the drive circuit in a case where the estimated temperature of the susceptor is higher than the target temperature, and an average value of the first voltage per unit time may be higher than an average value of the second voltage per the unit time.

The second voltage may be equal to 0.

To overcome the issue described above, another aspect of the present invention provides a program to be executed by a computer that controls an inhaler device, the inhaler device including: a power supply configured to supply DC electric power; a drive circuit including an inverter circuit configured to convert the DC electric power supplied from the power supply into AC electric power, and an electromagnetic induction source configured to generate a varying magnetic field by using the AC electric power supplied from the inverter circuit; a holder configured to hold a substrate including an aerosol source; and an electric power sensor configured to detect information of the DC electric power supplied from the power supply to the drive circuit, the electromagnetic induction source being disposed at a position where the varying magnetic field generated from the electromagnetic induction source penetrates a susceptor that is disposed in thermal proximity to the aerosol source included in the substrate held by the holder, the susceptor being configured to produce heat upon being penetrated by the varying magnetic field, the program causing controlling electric power supply to the drive circuit, based on information related to a disturbance and the information of the DC electric power supplied to the drive circuit detected by the electric power sensor to be performed.

To overcome the issue described above, another aspect of the present invention provides a system including: an inhaler device; and a substrate, the substrate including an aerosol source, the inhaler device including: a power supply configured to supply DC electric power; a drive circuit including an inverter circuit configured to convert the DC electric power supplied from the power supply into AC electric power, and an electromagnetic induction source configured to generate a varying magnetic field by using the AC electric power supplied from the inverter circuit; a controller configured to control electric power supply from the power supply to the drive circuit; a holder configured to hold the substrate; and an electric power sensor configured to detect information of the DC electric power supplied from the power supply to the drive circuit, in which the electromagnetic induction source is disposed at a position where the varying magnetic field generated from the electromagnetic induction source penetrates a susceptor that is disposed in thermal proximity to the aerosol source included in the substrate held by the holder, the susceptor is configured to produce heat upon being penetrated by the varying magnetic field, and the controller is configured to control the electric power supply to the drive circuit, based on information related to a disturbance and the information of the DC electric power supplied to the drive circuit detected by the electric power sensor.

The susceptor may be included in the substrate.

### Advantageous Effects of Invention

As described above, the present invention provides a mechanism that enables an inhaler device of induction heating type to appropriately generate an aerosol.

### Brief Description of Drawings

[Fig. 1] Fig. 1 is a schematic diagram of an inhaler device according to a configuration example.
[Fig. 2] Fig. 2 is a block diagram illustrating structural elements related to induction heating performed by the inhaler device according to the present embodiment.
[Fig. 3] Fig. 3 is a diagram illustrating an equivalent circuit of a circuit related to induction heating performed by the inhaler device according to the present embodiment.
[Fig. 4] Fig. 4 is a graph illustrating an example of a time-series change in an actual temperature of a susceptor heated by induction heating based on a heating profile presented by Table 1.
[Fig. 5] Fig. 5 is a diagram for describing a process of correcting an estimated temperature of a susceptor according to the present embodiment.
[Fig. 6] Fig. 6 is a diagram for describing a process of correcting an estimated temperature of a susceptor according to the present embodiment.
[Fig. 7] Fig. 7 is a diagram for describing an equivalent circuit of a circuit related to induction heating performed by the inhaler device according to the present embodiment, a relationship between an electrical resistance value and a temperature of a drive circuit, and a relationship between an electrical resistance value and a temperature of the susceptor.
[Fig. 8] Fig. 8 is a flowchart illustrating an example of a procedure of a process performed by the inhaler device according to the present embodiment.

### Description of Embodiments

A preferred embodiment of the present invention will be described in detail below with reference to the accompanying drawings. In the specification and the drawings, structural elements having substantially the same functional configuration are denoted by the same reference numerals, and redundant description thereof will be omitted.

### <1. Configuration example of inhaler device>

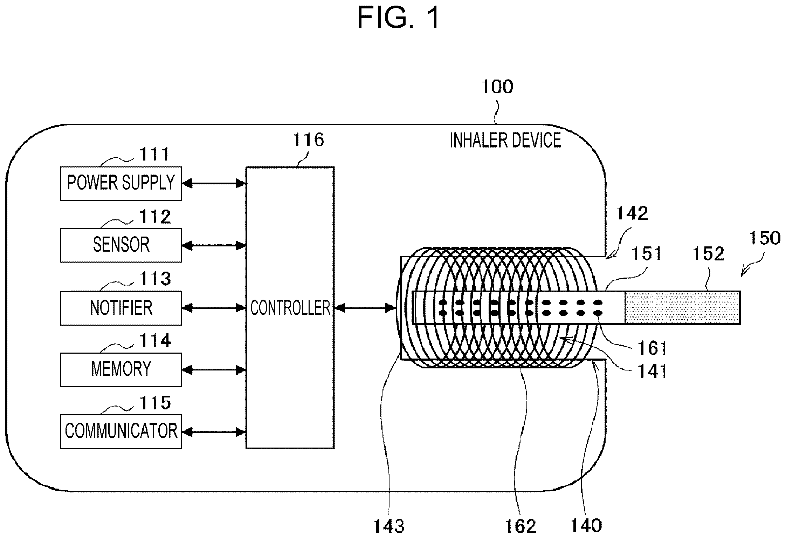

An inhaler device according to the present configuration example heats a substrate including an aerosol source by induction heating (IH) to generate an aerosol. The present configuration example will be described below with reference to Fig. 1.

Fig. 1 is a schematic diagram of the inhaler device according to the configuration example. As illustrated in Fig. 1, an inhaler device 100 according to the present configuration example includes a power supply 111, a sensor 112, a notifier 113, a memory 114, a communicator 115, a controller 116, a susceptor 161, an electromagnetic induction source 162, and a holder 140. A user performs inhalation while a stick substrate 150 is held by the holder 140. Each structural element will be sequentially described below.

The power supply 111 stores electric power. The power supply 111 supplies electric power to each structural element of the inhaler device 100. The power supply 111 may be, for example, a rechargeable battery such as a lithium ion secondary battery. The power supply 111 may be charged by being connected to an external power supply through a Universal Serial Bus (USB) cable or the like. In addition, the power supply 111 may be charged, by using a wireless power transmission technology, without being connected to a power-transmitting device. Further, the power supply 111 alone may be removed from the inhaler device 100 and replaced with a new power supply 111.

The sensor 112 detects various items of information regarding the inhaler device 100. The sensor 112 outputs the detected items of information to the controller 116. In an example, the sensor 112 may be a pressure sensor such as a condenser microphone, a flow sensor, or a temperature sensor. In response to detecting a numerical value in accordance with inhalation performed by a user, the sensor 112 outputs information indicating that the user has performed the inhalation to the controller 116. In another example, the sensor 112 may be an input device that accepts information input by the user, such as a button or a switch. In particular, the sensor 112 may include a button for inputting an instruction to start/stop generation of an aerosol. The sensor 112 outputs the information input by the user to the controller 116. In another example, the sensor 112 may be a temperature sensor that detects a temperature of the susceptor 161. The temperature sensor detects the temperature of the susceptor 161 based on, for example, an electrical resistance value of the electromagnetic induction source 162. The sensor 112 may detect the temperature of the stick substrate 150 held by the holder 140, based on the temperature of the susceptor 161.

The notifier 113 notifies the user of information. In an example, the notifier 113 may be a light-emitting device such as a light-emitting diode (LED). In this case, the notifier 113 emits different patterns of light when the power supply 111 needs to be charged, when the power supply 111 is being charged, when the inhaler device 100 has an anomaly, and so on. The pattern of light is a concept including a color, turn-on/turn-off timings, and so on. The notifier 113 may be, along with or instead of the light-emitting device, a display device that displays an image, a sound output device that outputs sound, or a vibration device that vibrates. In addition, the notifier 113 may notify the user of information indicating that the user can perform inhalation. The user is notified of the information indicating that the user can perform inhalation, in response to the temperature of the stick substrate 150 that produces heat by electromagnetic induction reaching a predetermined temperature.

The memory 114 stores various items of information for operation of the inhaler device 100. The memory 114 may be a non-volatile storage medium such as a flash memory. An example of the items of information stored in the memory 114 is items of information related to an operating system (OS) of the inhaler device 100, such as details of control performed on the various structural elements by the controller 116. Another example of the items of information stored in the memory 114 is items of information related to inhalation performed by the user, such as the number of times of inhalation, an inhalation time, and an accumulated inhalation time period.

The communicator 115 is a communication interface for transmitting and receiving information between the inhaler device 100 and another device. The communicator 115 performs communication in conformity with any wired or wireless communication standard. Such a communication standard may be, for example, a wireless local area network (LAN), a wired LAN, Wi-Fi (registered trademark), or Bluetooth (registered trademark). In an example, the communicator 115 transmits the items of information related to inhalation performed by the user to a smartphone to cause the smartphone to display the items of information related to inhalation performed by the user. In another example, the communicator 115 receives information of a new OS from a server to update the information of the OS stored in the memory 114.

The controller 116 functions as an arithmetic processing unit and a control circuit, and controls the overall operations of the inhaler device 100 in accordance with various programs. The controller 116 is implemented by an electronic circuit such as a central processing unit (CPU) or a microprocessor, for example. In addition, the controller 116 may include a read-only memory (ROM) that stores a program to be used, an arithmetic parameter, and the like, and a random access memory (RAM) that temporarily stores a parameter that changes as appropriate and the like. The inhaler device 100 performs various processes under the control of the controller 116. Electric power supply from the power supply 111 to each of the other structural elements, charging of the power supply 111, detection of information by the sensor 112, notification of information by the notifier 113, storage and reading of information to and from the memory 114, and transmission and reception of information by the communicator 115 are an example of the processes controlled by the controller 116. Other processes performed by the inhaler device 100, such as input of information to each structural element and a process based on information output from each structural element are also controlled by the controller 116.

The holder 140 has an internal space 141, and holds the stick substrate 150 in a manner such that the stick substrate 150 is partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 that is a bottom surface, and may define the pillar-shaped internal space 141. The holder 140 has, in at least a portion of the tubular body in the height direction, an inside diameter that is smaller than an outside diameter of the stick substrate 150 to be able to hold the stick substrate 150 by pressing the stick substrate 150 inserted into the internal space 141 from the outer circumference. The holder 140 also has a function of defining a flow path of air that passes through the stick substrate 150. For example, the bottom 143 has an air inlet hole that is an inlet of air into the flow path. On the other hand, the opening 142 serves as an air outlet hole that is an outlet of air from the flow path.

The stick substrate 150 is a stick-shaped member. The stick substrate 150 includes a substrate 151 and an inhalation port 152.

The substrate 151 includes an aerosol source. The aerosol source is heated to be atomized, so that an aerosol is generated. The aerosol source may be a material derived from tobacco, such as shredded tobacco or a processed material obtained by forming a tobacco raw material into a granular, sheet-like, or powdery shape. In addition, the aerosol source may include a material that is not derived from tobacco, such as a material made from a plant other than tobacco (for example, mint or an herb). In an example, the aerosol source may include a flavor component such as menthol. For the inhaler device 100 that is a medical inhaler, the aerosol source may include a medicine to be inhaled by a patient. The aerosol source is not limited to a solid and may be a liquid such as polyhydric alcohol and water. Examples of the polyhydric alcohol include glycerine and propylene glycol. At least a portion of the substrate 151 is accommodated in the internal space 141 of the holder 140 when the stick substrate 150 is held by the holder 140

The inhalation port 152 is to be held in a mouth of the user during inhalation. At least a portion of the inhalation port 152 protrudes from the opening 142 when the stick substrate 150 is held by the holder 140. When a user performs inhalation while holding, in their mouth, the inhalation port 152 protruding from the opening 142, air flows into the holder 140 through the air inlet hole (not illustrated). The air that has flowed in passes through the internal space 141 of the holder 140, that is, the substrate 151, and reaches the inside of the mouth of the user together with the aerosol generated from the substrate 151.

The stick substrate 150 further includes the susceptor 161. The susceptor 161 produces heat by electromagnetic induction. The susceptor 161 may be made of a conductive material such as metal. In an example, the susceptor 161 is a piece of metal. The susceptor 161 is disposed in proximity to the aerosol source. In the example illustrated in Fig. 1, the susceptor 161 is included in the substrate 151 of the stick substrate 150.

The susceptor 161 is disposed in thermal proximity to the aerosol source. The susceptor 161 being in thermal proximity to the aerosol source means that the susceptor 161 is disposed at a position where heat produced by the susceptor 161 is transferred to the aerosol source. For example, the susceptor 161 is included in the substrate 151 along with the aerosol source and is surrounded by the aerosol source. This configuration enables the heat produced by the susceptor 161 to be efficiently used for heating the aerosol source.

Note that the susceptor 161 may be untouchable from outside of the stick substrate 150. For example, the susceptor 161 may be distributed in a central part of the stick substrate 150, but does not have to be distributed near the outer circumference of the stick substrate 150.

The electromagnetic induction source 162 causes the susceptor 161 to produce heat by electromagnetic induction. For example, the electromagnetic induction source 162 is a coiled conductive wire wound around the outer circumference of the holder 140. Upon being supplied with an alternating current from the power supply 111, the electromagnetic induction source 162 generates a magnetic field. The electromagnetic induction source 162 is disposed at a position where the internal space 141 of the holder 140 overlaps with the generated magnetic field. Thus, when a magnetic field is generated while the stick substrate 150 is held by the holder 140, an eddy current is generated in the susceptor 161 to generate Joule heat. The aerosol source included in the stick substrate 150 is heated by the Joule heat to be atomized, so that an aerosol is generated. In an example, when the sensor 112 detects a predetermined user input, electric power may be supplied and an aerosol may be generated. When the temperature of the stick substrate 150 that is heated by induction heating using the susceptor 161 and the electromagnetic induction source 162 reaches a predetermined temperature, the user can perform inhalation. When the sensor 112 detects a predetermined user input thereafter, electric power supply may be stopped. In another example, electric power may be supplied and an aerosol may be generated, while the sensor 112 detects inhalation performed by the user.

Fig. 1 illustrates an example of the susceptor 161 included in the substrate 151 of the stick substrate 150. However, the present configuration example is not limited to such an example. For example, the holder 140 may function as the susceptor 161. In this case, the magnetic field generated by the electromagnetic induction source 162 generates an eddy current in the holder 140, so that Joule heat is generated. The aerosol source included in the stick substrate 150 is heated by the Joule heat to be atomized, so that an aerosol is generated.

The combination of the inhaler device 100 and the stick substrate 150 may be regarded as a single system because an aerosol can be generated by combining the inhaler device 100 and the stick substrate 150.

### <2. Induction heating>

Induction heating will be described in detail below.

Induction heating is a process of heating a conductive object by causing a varying magnetic field to penetrate the object. Induction heating involves a magnetic field generator that generates a varying magnetic field, and a to-be-heated object that is conductive and is to be heated when exposed to the varying magnetic field. An example of the varying magnetic field is an alternating magnetic field. The electromagnetic induction source 162 illustrated in Fig. 1 is an example of the magnetic field generator. The susceptor 161 illustrated in Fig. 1 is an example of the to-be-heated object.

The magnetic field generator and the to-be-heated object are disposed at relative positions such that a varying magnetic field generated from the magnetic field generator penetrates the to-be-heated object. When a varying magnetic field is generated from the magnetic field generator in this state, an eddy current is induced in the to-be-heated object. The eddy current flows through the to-be-heated object, which produces Joule heat according to the electrical resistance of the to-be-heated object, so that the to-be-heated object is heated. Such heating is also referred to as Joule heating, ohmic heating, or resistive heating.

The to-be-heated object may be magnetic. In this case, the to-be-heated object is further heated by magnetic hysteresis heating. Magnetic hysteresis heating is a process of heating a magnetic object by causing a varying magnetic field to penetrate the object. When a magnetic field penetrates a magnetic body, magnetic dipoles included in the magnetic body are aligned along the magnetic field. Thus, when a varying magnetic field penetrates a magnetic body, the orientation of the magnetic dipoles changes in accordance with the applied varying magnetic field. Such reorientation of the magnetic dipoles produces heat in the magnetic body, so that the to-be-heated object is heated.

Magnetic hysteresis heating typically occurs at a temperature of the Curie point or lower and does not occur at a temperature exceeding the Curie point. The Curie point is the temperature at which a magnetic body loses magnetic properties thereof. For example, when the temperature of a to-be-heated object that is ferromagnetic at a temperature of the Curie point or lower exceeds the Curie point, a reversible phase transition from ferromagnetism to paramagnetism occurs in the magnetism of the to-be-heated object. When the temperature of the to-be-heated object exceeds the Curie point, magnetic hysteresis heating no longer occurs. Thus, the temperature increase rate slows down.

The to-be-heated object is desirably made of a conductive material. Further, the to-be-heated object is desirably made of a ferromagnetic material. This is because the combination of resistive heating and magnetic hysteresis heating can increase the heating efficiency in the latter case. For example, the to-be-heated object may be made of one or more materials selected from a material group including aluminum, iron, nickel, cobalt, conductive carbon, copper, and stainless steel.

In both resistive heating and magnetic hysteresis heating, heat is produced inside the to-be-heated object rather than by thermal conduction from an external heat source. This thus can implement a rapid temperature increase and a uniform heat distribution in the to-be-heated object. This can be implemented by appropriately designing the material and shape of the to-be-heated object and the magnitude and direction of the varying magnetic field. That is, a rapid temperature increase and a uniform heat distribution can be implemented in the stick substrate 150 by appropriately designing the distribution of the susceptor 161 included in the stick substrate 150. This thus can reduce the time for preheating and improve the quality of a flavor tasted by the user.

Since induction heating directly heats the susceptor 161 included in the stick substrate 150, the substrate can be heated more efficiently than when the stick substrate 150 is heated from the outer circumference or the like by an external heat source. When heating is performed using an external heat source, the temperature of the external heat source inevitably becomes higher than that of the stick substrate 150. In contrast, when induction heating is performed, the temperature of the electromagnetic induction source 162 does not become higher than that of the stick substrate 150. Thus, the temperature of the inhaler device 100 can be maintained to be lower than that in the case of using an external heat source. This is a great advantage in terms of user safety.

The electromagnetic induction source 162 generates a varying magnetic field by using electric power supplied from the power supply 111. In an example, the power supply 111 may be a direct current (DC) power supply. In this case, the power supply 111 supplies, via a DC/altemate current (AC) inverter, AC electric power to the electromagnetic induction source 162. In this case, the electromagnetic induction source 162 can generate an alternating magnetic field.

The electromagnetic induction source 162 is disposed at a position where the varying magnetic field generated from the electromagnetic induction source 162 penetrates the susceptor 161 disposed in thermal proximity to the aerosol source included in the stick substrate 150 held by the holder 140. The susceptor 161 produces heat upon being penetrated by the varying magnetic field. The electromagnetic induction source 162 illustrated in Fig. 1 is a solenoid coil. The solenoid coil is disposed such that the conductive wire is wound around the outer circumference of the holder 140. When a current is applied to the solenoid coil, a magnetic field is generated in a central space surrounded by the coil, that is, the internal space 141 of the holder 140. As illustrated in Fig. 1, the susceptor 161 is surrounded by the coil when the stick substrate 150 is held by the holder 140. Thus, the varying magnetic field generated from the electromagnetic induction source 162 penetrates the susceptor 161 and heats the susceptor 161 by induction heating.

### <3. Technical features>

### (1) Detailed internal configuration

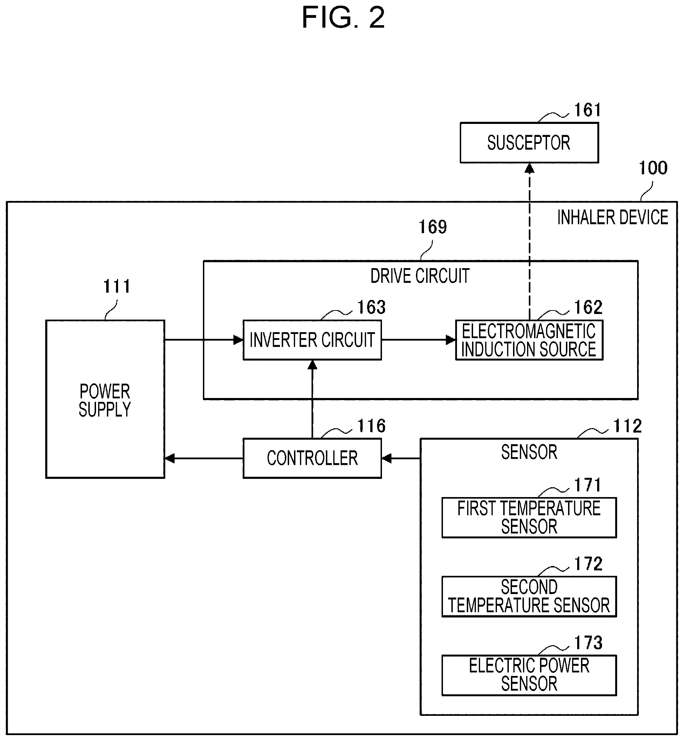

Structural elements related to induction heating according to the present embodiment will be described in detail with reference to Fig. 2. Fig. 2 is a block diagram illustrating structural elements related to induction heating performed by the inhaler device 100 according to the present embodiment.

As illustrated in Fig. 2, the inhaler device 100 includes a drive circuit 169 including the electromagnetic induction source 162 and an inverter circuit 163. The drive circuit 169 is a circuit for generating a varying magnetic field. The drive circuit 169 may further include another circuit such as a matching circuit. The drive circuit 169 operates by using electric power supplied from the power supply 111.

The power supply 111 is a DC power supply and supplies DC electric power. The inverter circuit 163 converts the DC electric power supplied from the power supply 111 into AC electric power. The inverter circuit 163 includes at least one switching element, and generates AC electric power by switching the switching element ON and OFF. The electromagnetic induction source 162 generates a varying magnetic field by using the AC electric power supplied from the inverter circuit 163. When the varying magnetic field generated from the electromagnetic induction source 162 penetrates the susceptor 161, the susceptor 161 produces heat.

As illustrated in Fig. 2, the sensor 112 includes a first temperature sensor 171, a second temperature sensor 172, and an electric power sensor 173.

The first temperature sensor 171 detects a temperature of an external environment. The temperature of the external environment is an ambient temperature. The first temperature sensor 171 is disposed at a position separate from the holder 140 to avoid an influence of induction heating. In an example, the first temperature sensor 171 may be disposed in proximity to a housing that is the outermost shell of the inhaler device 100.

The second temperature sensor 172 detects a temperature of the drive circuit 169. The second temperature sensor 172 is disposed in proximity to the drive circuit 169.

The electric power sensor 173 detects information of the DC electric power supplied from the power supply 111 to the drive circuit 169. The information of the DC electric power may be a current value and a voltage value. In an example, the electric power sensor 173 may include a micro controller unit (MCU) having a feedback channel from the power supply 111. The electric power sensor 173 detects a current value and a voltage value of the DC electric power supplied to the drive circuit 169, based on the feedback from the power supply 111.

The controller 116 controls electric power supply from the power supply 111 to the drive circuit 169. In particular, the controller 116 controls electric power supply to the drive circuit 169, based on the information of the DC electric power supplied to the drive circuit 169, which is detected by the electric power sensor 173. Specifically, first, the controller 116 estimates the temperature of the susceptor 161, based on the information of the DC electric power supplied to the drive circuit 169. The controller 116 then controls electric power supply to the drive circuit 169, based on the estimated temperature of the susceptor 161. For example, the controller 116 controls electric power supply to the drive circuit 169 such that the temperature of the susceptor 161 changes in accordance with a heating profile described later. Alternatively, the controller 116 may control the operation of the inverter circuit 163 such as controlling ON/OFF timings of the switching element.

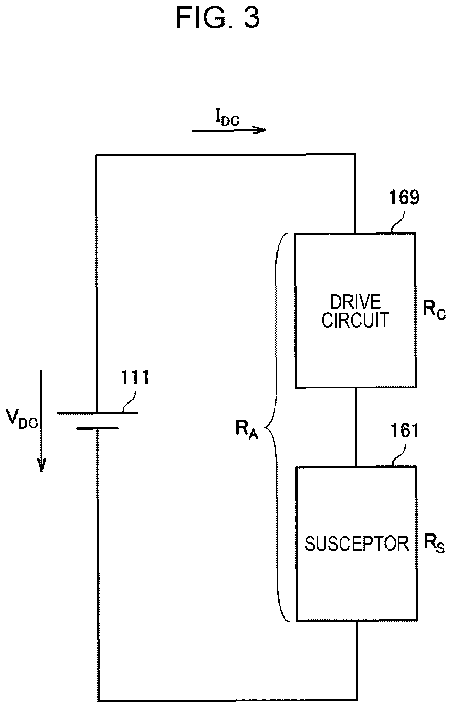

Patent Literature 1 above discloses details of the method of estimating the temperature of the susceptor 161. The method of estimating the temperature of the susceptor 161 will be briefly described with reference to Fig. 3.

Fig. 3 is a diagram illustrating an equivalent circuit of a circuit related to induction heating performed by the inhaler device 100 according to the present embodiment. An apparent electrical resistance value R_{A} illustrated in Fig. 3 is an electrical resistance value of a closed circuit including the drive circuit 169, calculated from a current value I_{DC} and a voltage value V_{DC} of the DC electric power supplied from the power supply 111 to the drive circuit 169. As illustrated in Fig. 3, the apparent electrical resistance value R_{A} corresponds to a series connection formed by an electrical resistance value R_{C} of the drive circuit 169 and an electrical resistance value Rs of the susceptor 161. The apparent electrical resistance value R_{A} and the temperature of the susceptor 161 have a very monotonic relationship therebetween. For example, the apparent electrical resistance value R_{A} and the temperature of the susceptor 161 have a substantially linear relationship therebetween within a range (for example, from 0°C to 400°C) in which the temperature of the susceptor 161 may change due to induction heating performed by the inhaler device 100. Thus, the controller 116 can calculate the apparent electrical resistance value R_{A}, based on the current value I_{DC} and the voltage value V_{DC}, and estimate the temperature of the susceptor 161, based on the apparent electrical resistance value R_{A}.

### (2) Heating profile

The controller 116 controls electric power supply from the power supply 111 to the drive circuit 169 based on a heating profile. The controller 116 controls DC electric power supplied from the power supply 111 to the drive circuit 169 to control AC electric power supplied from the inverter circuit 163 to the electromagnetic induction source 162 and control the operation of the electromagnetic induction source 162.

The heating profile is information that defines a time-series change in a target temperature that is a target value of the temperature of the susceptor 161. The controller 116 controls electric power supply to the drive circuit 169 such that a real temperature (hereinafter, also referred to as an actual temperature) of the susceptor 161 changes in the same manner as the time-series change in the target temperature defined in the heating profile. Consequently, an aerosol is generated as planned in the heating profile. The heating profile is typically designed to optimize a flavor tasted by a user when the user inhales the aerosol generated from the stick substrate 150. Thus, by controlling electric power supply to the drive circuit 169 based on the heating profile, the flavor tasted by the user can be optimized.

The heating profile includes one or more combinations of an elapsed time from the start of heating and a target temperature to be reached at the elapsed time. The controller 116 controls the temperature of the susceptor 161, based on a deviation of the current actual temperature from the target temperature corresponding to the current elapsed time from the start of heating in the heating profile. Control of the temperature of the susceptor 161 can be implemented by known feedback control, for example. In the feedback control, the controller 116 may control electric power to be supplied to the drive circuit 169, based on a difference between the actual temperature and the target temperature or the like. The feedback control may be, for example, a proportional-integral-differential controller (PID controller). Alternatively, the controller 116 may simply perform ON-OFF control. For example, the controller 116 may supply electric power to the drive circuit 169 until the actual temperature reaches the target temperature, and may interrupt electric power supply to the drive circuit 169 upon the actual temperature reaching the target temperature.

The controller 116 controls a voltage to be applied to the drive circuit 169 to control electric power supply from the power supply 111 to the drive circuit 169. By controlling the voltage to be applied to the drive circuit 169, the controller 116 controls a magnetic force of the varying magnetic field generated from the electromagnetic induction source 162. In this way, the controller 116 can control the amount of heat produced by the susceptor 161.

If the estimated temperature of the susceptor 161 is lower than the target temperature, the controller 116 may perform control to apply a first voltage to the drive circuit 169. On the other hand, if the estimated temperature of the susceptor 161 is higher than the target temperature, the controller 116 may perform control to apply a second voltage to the drive circuit 169. In this case, an average value of the first voltage per unit time is greater than an average value of the second voltage per unit time. With such a configuration, when the estimated temperature of the susceptor 161 is lower than the target temperature, the temperature of the susceptor 161 can be increased to reach the target temperature. In addition, when the estimated temperature of the susceptor 161 is higher than the target temperature, the temperature of the susceptor 161 can be decreased to reach the target temperature.

There are various methods for controlling the average value of the voltage per unit time. In an example, the controller 116 may cause the power supply 111 to apply, to the drive circuit 169, a pulsed voltage according to pulse width modulation (PWM). In this case, the controller 116 may control the voltage per unit time by adjusting a duty ratio of an electric power pulse. Specifically, the duty ratio in the section in which the first voltage is applied may be larger than the duty ratio in the section in which the second voltage is applied. In another example, the controller 116 may control the voltage per unit time by increasing or decreasing the voltage itself. In addition, the controller 116 may increase or decrease the temperature of the susceptor 161 by controlling a frequency of the inverter circuit 163. For example, the controller 116 may make the frequency of the inverter circuit 163 match the frequency of a resonance circuit to implement efficient heating and increase the temperature of the susceptor 161. On the other hand, the controller 116 may make the frequency of the inverter circuit 163 different from the frequency of the resonance circuit to make heating inefficient and decrease the temperature of the susceptor 161.

Note that the second voltage may be equal to 0. That is, the controller 116 may stop electric power supply to the drive circuit 169. With such a configuration, the temperature of the susceptor 161 can be decreased most quickly.

A time section from the start to the end of a process of generating an aerosol by using the stick substrate 150, more specifically, a time section in which the electromagnetic induction source 162 operates based on the heating profile, is also referred to as a heating session hereinafter. The start of the heating session is a timing at which heating based on the heating profile is started. The end of the heating session is a timing at which a sufficient amount of aerosol is no longer generated. The heating session is constituted by a preheating period which is a first part and a puffable period which is a latter part. The puffable period is a period in which a sufficient amount of aerosol is expected to be generated. The preheating period is a period from the start of heating to the start of the puffable period. Heating performed in the preheating period is also referred to as preheating.

Table 1 below presents an example of the heating profile.

### [Table 1]

**Table 1_{.} Example of heating profile**

| Time section | Elapsed time from start of heating | Target temperature |
|---|---|---|
| Initial temperature rise section | 25 s | 295°C |
| | 35 s | 295°C |
| Intermediate temperature drop section | 45 s | 230°C |
| Temperature re-rise section | 180 s | 230°C |
| | 260 s | 260°C |
| | 355 s | 260°C |
| Heating termination section | Thereafter | - |

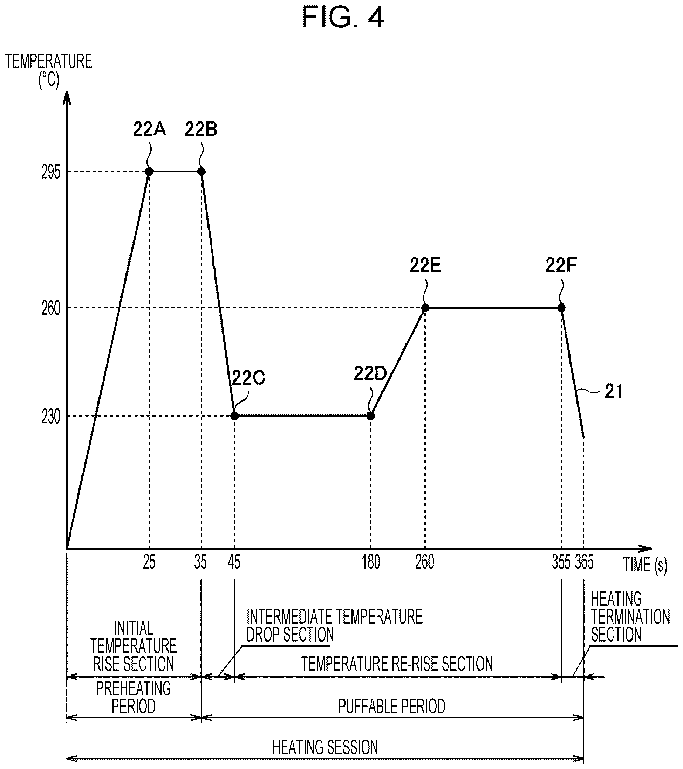

A time-series change in the actual temperature of the susceptor 161 when the controller 116 controls electric power supply to the drive circuit 169 in accordance with the heating profile presented by Table 1 will be described with reference to Fig. 4. Fig. 4 is a graph illustrating an example of a time-series change in the actual temperature of the susceptor 161 heated by induction heating based on the heating profile presented by Table 1. The horizontal axis of this graph represents time (seconds). The vertical axis of the graph represents the temperature of the susceptor 161. A line 21 in this graph represents a time-series change in the actual temperature of the susceptor 161. Points 22 (22A to 22F) in this graph each correspond to a target temperature defined in the heating profile. As illustrated in Fig. 4, the actual temperature of the susceptor 161 changes in the same manner as the time-series change in the target temperature defined in the heating profile.

As presented by Table 1, the heating profile first includes an initial temperature rise section. The initial temperature rise section is a time section included at the beginning of the heating profile, and is a section in which the target temperature set at the end of the section is higher than an initial temperature. The initial temperature is a temperature expected as the temperature of the susceptor 161 before heating is started. An example of the initial temperature is any temperature such as 0°C. Another example of the initial temperature is a temperature corresponding to an ambient temperature. As illustrated in Fig. 4, according to the target temperature set in the initial temperature rise section, the actual temperature of the susceptor 161 reaches 295°C after 25 seconds from the start of heating, and is maintained at 295°C until after 35 seconds from the start of heating. Accordingly, the temperature of the stick substrate 150 is expected to reach a temperature at which a sufficient amount of aerosol is to be generated. Since the actual temperature quickly rises to 295°C immediately after the start of heating, preheating can be finished early and the puffable period can be started early. Fig. 4 illustrates an example in which the initial temperature rise section coincides with the preheating period. However, the initial temperature rise section and the preheating period may differ from each other.

As presented by Table 1, the heating profile next includes an intermediate temperature drop section. The intermediate temperature drop section is a time section after the initial temperature rise section, and is a time section in which the target temperature set at the end of the time section is lower than the target temperature set at the end of the initial temperature rise section. As illustrated in Fig. 4, according to the target temperature set in the intermediate temperature drop section, the actual temperature of the susceptor 161 drops from 295°C to 230°C from 35 seconds to 45 seconds after the start of heating. In this section, electric power supply to the drive circuit 169 may be stopped. Even in such a case, a sufficient amount of aerosol is generated by residual heat of the susceptor 161 and the stick substrate 150. If the susceptor 161 is maintained at a high temperature, the aerosol source included in the stick substrate 150 is rapidly consumed. This may cause inconvenience that a flavor tasted by the user becomes too strong. However, by providing the intermediate temperature drop section in midstream, such inconvenience can be avoided and the quality of the user's puff experience can be improved.

As presented by Table 1, the heating profile next includes a temperature re-rise section. The temperature re-rise section is a time section after the intermediate temperature drop section, and is a time section in which the target temperature set at the end of the time section is higher than the target temperature set at the end of the intermediate temperature drop section. As illustrated in Fig. 4, according to the target temperature set in the temperature re-rise section, the actual temperature of the susceptor 161 increases stepwise from 230°C to 260°C from 45 seconds to 355 seconds after the start of heating. If the temperature of the susceptor 161 is continuously decreased, the temperature of the stick substrate 150 also decreases. Thus, the amount of generated aerosol decreases, and the flavor tasted by the user may deteriorate. However, by causing the actual temperature to re-rise after dropping, deterioration of the flavor tasted by the user can be prevented even in the latter part of the heating session.

As presented by Table 1, the heating profile lastly includes a heating termination section. The heating termination section is a time section after the temperature re-rise section, and is a time section in which heating is not performed. No target temperature may be set. As illustrated in Fig. 4, the actual temperature of the susceptor 161 drops after 355 seconds from the start of heating. Electric power supply to the drive circuit 169 may be terminated after 355 seconds from the start of heating. Even in such a case, a sufficient amount of aerosol is generated for a while by residual heat of the susceptor 161 and the stick substrate 150. In the example illustrated in Fig. 4, the puffable period, that is, the heating session ends after 365 seconds from the start of heating.

The user may be notified of the start timing and the end timing of the puffable period. The user may also be notified of a timing that is before the end of the puffable period by a predetermined time (for example, the end timing of the temperature re-rise section). In this case, the user can perform a puff in the puffable period with reference to the notification.

### (3) Control of electric power supply based on information related to disturbance

The controller 116 controls electric power supply to the drive circuit 169, based on information related to a disturbance and the information of the DC electric power supplied to the drive circuit 169 detected by the electric power sensor 173. The disturbance refers to a factor that influences the temperature of the susceptor 161. The controller 116 estimates the temperature of the susceptor 161, based on the information related to the disturbance and information of the DC electric power supplied to the drive circuit 169. Specifically, the controller 116 corrects, based on the information related to the disturbance, the temperature of the susceptor 161 estimated based on the information of the DC electric power supplied to the drive circuit 169. The controller 116 then controls electric power supply to the drive circuit 169, based on the estimated temperature of the susceptor 161. Such a configuration enables electric power supply to be controlled in consideration of the disturbance that influences the temperature of the susceptor 161. Thus, appropriate generation of an aerosol can be implemented. Specific disturbances and details of control corresponding to each of the disturbances will be described in detail below.

### - Control of electric power supply based on temperature of external environment

The information related to the disturbance may include the temperature of the external environment. That is, the controller 116 controls electric power supply to the drive circuit 169, based on the temperature of the external environment detected by the first temperature sensor 171 and the information of the DC electric power supplied to the drive circuit 169 detected by the electric power sensor 173. In environments in which the temperature of the external environment is different, the temperature of the susceptor 161 may be different even for the same apparent electrical resistance value R_{A}. However, the above configuration enables electric power supply to be controlled in consideration of the temperature of the external environment that influences the temperature of the susceptor 161. Thus, appropriate generation of an aerosol can be implemented.

The controller 116 estimates the temperature of the susceptor 161, based on the temperature of the external environment and the information of the DC electric power supplied to the drive circuit 169, and controls electric power supply to the drive circuit 169, based on the estimated temperature of the susceptor 161. With such a configuration, the temperature of the susceptor 161 can be more accurately estimated than in the case where the temperature of the external environment is not taken into account. Then, the temperature of the susceptor 161 can be controlled based on the more accurately estimated temperature of the susceptor 161. Thus, appropriate generation of an aerosol can be implemented.

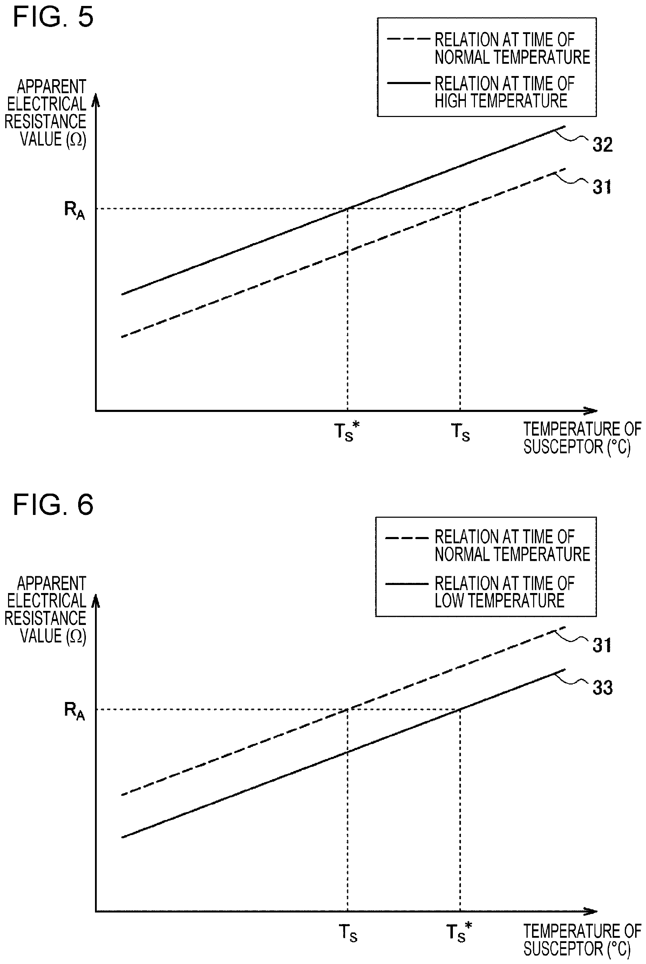

Specifically, the controller 116 corrects, based on the temperature of the external environment, the temperature of the susceptor 161 estimated based on the information of the DC electric power supplied to the drive circuit 169. That is, the controller 116 corrects, based on the temperature of the external environment, the temperature of the susceptor 161 estimated based on the apparent electrical resistance value R_{A}. With such a configuration, the temperature of the susceptor 161 can be more accurately estimated by correcting an estimation error in the temperature of the susceptor 161 due to the temperature of the external environment. The correction will be described with reference to Figs. 5 and 6.

Fig. 5 is a diagram for describing a process of correcting the estimated temperature of the susceptor 161 according to the present embodiment. Fig. 5 illustrates correction to be performed when the temperature of the external environment is higher than a first reference temperature. Note that the first reference temperature is a temperature at which an estimation error of the temperature of the susceptor 161 estimated based on the apparent electrical resistance value R_{A} falls within an allowable range when the temperature of the external environment is lower than or equal to the first reference temperature. A line 31 represents a relation at the time of normal temperature, which established between the apparent electrical resistance value R_{A} and the temperature of the susceptor 161 when the temperature of the external environment is a standard temperature. A line 32 represents a relation at the time of high temperature, which is established between the apparent electrical resistance value R_{A} and the temperature of the susceptor 161 when the temperature of the external environment is higher than the first reference temperature.

In an example, the controller 116 estimates that the temperature of the susceptor 161 is Ts, based on the relation 31 at the time of normal temperature and the apparent electrical resistance value R_{A} that is calculated based on the information of the DC electric power supplied to the drive circuit 169. If the temperature of the external environment is higher than the first reference temperature, the controller 116 estimates that the temperature of the susceptor 161 is Ts` that is lower than Ts.

In another example, when the temperature of the external environment is higher than the first reference temperature, the controller 116 estimates that the temperature of the susceptor 161 is Ts`, based on the relation 32 at the time of high temperature and the apparent electrical resistance value R_{A} that is calculated based on the information of the DC electric power supplied to the drive circuit 169.

The temperature of the susceptor 161 can be estimated based on the apparent electrical resistance value R_{A} by using the fact that the apparent electrical resistance value R_{A} changes in accordance with a change in the temperature of the susceptor 161. However, if the temperature of the external environment is high, the temperature of the susceptor 161 and/or the temperature of the drive circuit 169 increase(s), and the apparent electrical resistance value R_{A} increases accordingly. In this case, the temperature of the susceptor 161 estimated based only on the apparent electrical resistance value R_{A} becomes higher than the actual temperature of the susceptor 161. However, by performing the correction described above with reference to Fig. 5, the estimation error caused by the temperature of the external environment can be corrected and the temperature of the susceptor 161 can be more accurately estimated.

Fig. 6 is a diagram for describing a process of correcting the estimated temperature of the susceptor 161 according to the present embodiment. Fig. 6 illustrates correction to be performed when the temperature of the external environment is lower than a second reference temperature. Note that the second reference temperature is a temperature at which an estimation error of the temperature of the susceptor 161 estimated based on the apparent electrical resistance value R_{A} falls within an allowable range when the temperature of the external environment is higher than or equal to the second reference temperature. The first reference temperature and the second reference temperature may be the same or may be different. The line 31 represents the relation at the time of normal temperature, which established between the apparent electrical resistance value R_{A} and the temperature of the susceptor 161 when the temperature of the external environment is the standard temperature. A line 33 represents a relation at the time of low temperature, which is established between the apparent electrical resistance value R_{A} and the temperature of the susceptor 161 when the temperature of the external environment is lower than the second reference temperature.

In an example, the controller 116 estimates that the temperature of the susceptor 161 is Ts, based on the relation 31 at the time of normal temperature and the apparent electrical resistance value R_{A} that is calculated based on the information of the DC electric power supplied to the drive circuit 169. If the temperature of the external environment is lower than the second reference temperature, the controller 116 estimates that the temperature of the susceptor 161 is Ts` that is higher than Ts.

In another example, when the temperature of the external environment is lower than the second reference temperature, the controller 116 estimates that the temperature of the susceptor 161 is Ts`, based on the relation 33 at the time of low temperature and the apparent electrical resistance value R_{A} that is calculated based on the information of the DC electric power supplied to the drive circuit 169.

The temperature of the susceptor 161 can be estimated based on the apparent electrical resistance value R_{A} by using the fact that the apparent electrical resistance value R_{A} changes in accordance with a change in the temperature of the susceptor 161. However, if the temperature of the external environment is low, the temperature of the susceptor 161 and/or the temperature of the drive circuit 169 decrease(s), and the apparent electrical resistance value R_{A} decreases accordingly. In this case, the temperature of the susceptor 161 estimated based only on the apparent electrical resistance value R_{A} becomes lower than the actual temperature of the susceptor 161. However, by performing the correction described above with reference to Fig. 6, the estimation error caused by the temperature of the external environment can be corrected and the temperature of the susceptor 161 can be more accurately estimated.

When performing the correction, the controller 116 corrects the temperature of the susceptor estimated based on the information of the DC electric power supplied to the drive circuit 169, to be lower as the temperature of the external environment is higher and to be higher as the temperature of the external environment is lower. In the example illustrated in Fig. 5, as the difference between the temperature of the external environment and the first reference temperature increases, the difference between the temperature Ts of the susceptor 161 before the correction and the temperature Ts` of the susceptor 161 after the correction increases. In the example illustrated in Fig. 6, as the difference between the temperature of the external environment and the second reference temperature increases, the difference between the temperature Ts of the susceptor 161 before the correction and the temperature Ts* of the susceptor 161 after the correction increases. With such a configuration, the temperature of the susceptor 161 can be more accurately estimated by correcting an estimation error in the temperature of the susceptor 161 due to the temperature of the external environment.

### -Control of electric power supply based on temperature of drive circuit 169

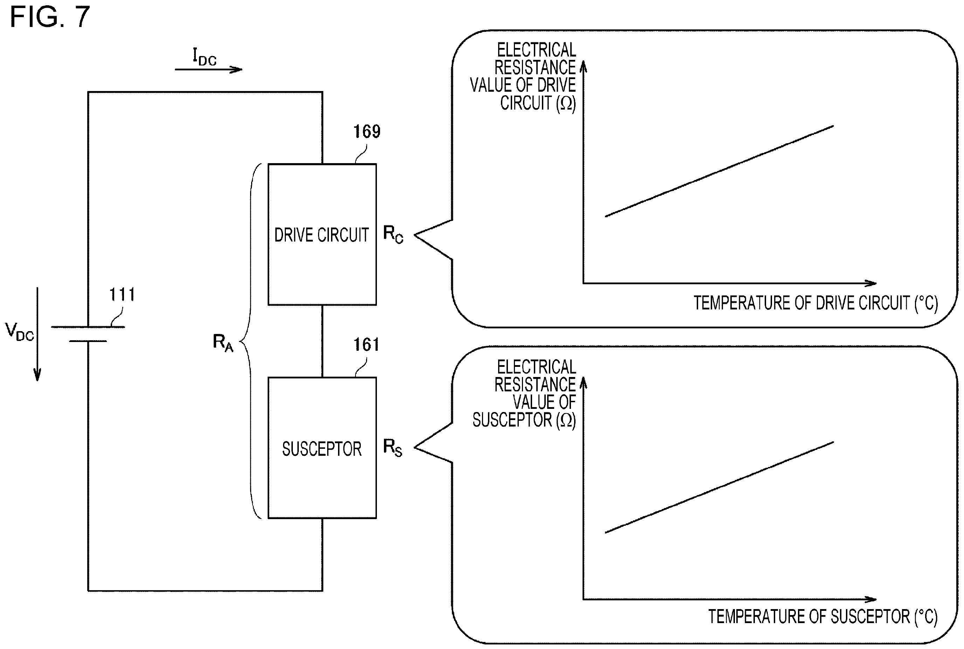

Fig. 7 is a diagram for describing an equivalent circuit of a circuit related to induction heating performed by the inhaler device 100 according to the present embodiment, a relationship between the electrical resistance value and the temperature of the drive circuit 169, and a relationship between the electrical resistance value and the temperature of the susceptor 161. As illustrated in Fig. 7, the electrical resistance value Rs of the susceptor 161 changes in accordance with a change in the temperature of the susceptor 161. Likewise, the electrical resistance value R_{C} of the drive circuit 169 changes in accordance with a change in the temperature of the drive circuit 169. That is, when electric power is supplied to the drive circuit 169, the susceptor 161 produces heat and the electromagnetic induction source 162 may also produce heat. Therefore, the apparent electrical resistance value R_{A} is influenced not only by the change in the electrical resistance value Rs due to the change in the temperature of the susceptor 161 but also by the change in the electrical resistance value R_{C} due to the change in the temperature of the drive circuit 169.

Thus, the information related to the disturbance may include the temperature of the drive circuit 169. In other words, the information related to the disturbance may include the electrical resistance value R_{C} of the drive circuit 169. That is, the controller 116 may control electric power supply to the drive circuit 169, further based on the electrical resistance value Rs of the drive circuit 169. With such a configuration, electric power supply can be controlled in consideration of the change in the electrical resistance value R_{S} due to the change in the temperature of the drive circuit 169, which may cause an estimation error in the temperature of the susceptor 161. Thus, appropriate generation of an aerosol can be implemented.

The controller 116 may estimate the electrical resistance value Rs of the drive circuit 169, based on the temperature of the drive circuit 169 detected by the second temperature sensor 172. Then, the controller 116 may control electric power supply to the drive circuit 169, based on the estimated electrical resistance value R_{S} of the drive circuit 169. With such a configuration, electric power supply to the drive circuit 169 can be controlled based on a real-time change in the electrical resistance value Rs of the drive circuit 169, which corresponds to a real-time change in the temperature of the drive circuit 169.

The controller 116 may estimate the temperature of the susceptor 161, based on the electrical resistance value Rs of the drive circuit 169, and control electric power supply to the drive circuit 169, based on the estimated temperature of the susceptor 161. With such a configuration, the temperature of the susceptor 161 can be more accurately estimated than in the case where the temperature of the drive circuit 169 is not taken into account. Then, the temperature of the susceptor 161 can be controlled based on the more accurately estimated temperature of the susceptor 161. Thus, appropriate generation of an aerosol can be implemented.

Specifically, the controller 116 may correct, based on the electrical resistance value Rs of the drive circuit 169, the temperature of the susceptor 161 estimated based on the information of the DC electric power supplied to the drive circuit 169. That is, the controller 116 may correct, based on the electrical resistance value Rs of the drive circuit 169, the temperature of the susceptor 161 estimated based on the apparent electrical resistance value R_{A}. With such a configuration, the temperature of the susceptor 161 can be more accurately estimated by correcting an estimation error in the temperature of the susceptor 161 due to the electrical resistance value R_{S} of the drive circuit 169.

The controller 116 may correct the temperature of the susceptor 161 estimated based on the information of the DC electric power supplied to the drive circuit 169, to be lower as the electrical resistance value Rs of the drive circuit 169 is larger. When the temperature of the drive circuit 169 increases, the electrical resistance value Rs of the drive circuit 169 increases and thus the apparent electrical resistance value R_{A} increases. In this case, the temperature of the susceptor 161 estimated based only on the apparent electrical resistance value R_{A} becomes higher than the actual temperature of the susceptor 161. However, by estimating the temperature of the susceptor 161 to be lower as the electrical resistance value Rs of the drive circuit 169 is larger, the estimation error caused by the temperature of the drive circuit 169 can be corrected and the temperature of the susceptor 161 can be estimated more accurately.

On the other hand, the controller 116 corrects the temperature of the susceptor 161 estimated based on the information of the DC electric power supplied to the drive circuit 169, to be higher as the electrical resistance value Rs of the drive circuit 169 is smaller. When the temperature of the drive circuit 169 decreases, the electrical resistance value Rs of the drive circuit 169 decreases and thus the apparent electrical resistance value R_{A} decreases. In this case, the temperature of the susceptor 161 estimated based only on the apparent electrical resistance value R_{A} becomes lower than the actual temperature of the susceptor 161. However, by estimating the temperature of the susceptor 161 to be higher as the electrical resistance value Rs of the drive circuit 169 is smaller, the estimation error caused by the temperature of the drive circuit 169 can be corrected and the temperature of the susceptor 161 can be estimated more accurately.

Note that the controller 116 may obviously control electric power supply to the drive circuit 169, based on both the temperature of the external environment and the electrical resistance value Rs of the drive circuit 169. In addition, the controller 116 may operate in any of first to third operation modes described below. The first operation mode is an operation mode in which electric power supply is controlled based on the temperature of the external environment. The second operation mode is an operation mode in which electric power supply is control based on the temperature of the drive circuit 169. The third operation mode is an operation mode in which electric power supply is controlled based on both the temperature of the external environment and the temperature of the drive circuit 169. The operation mode in which the controller 116 operates can be switched based on various situations such as the temperature of the external environment, the temperature of the drive circuit 169, and the remaining amount of electric power of the power supply 111, for example. With such a configuration, the controller 116 can operate in an operation mode suitable for a situation.

### (4) Procedure of Process

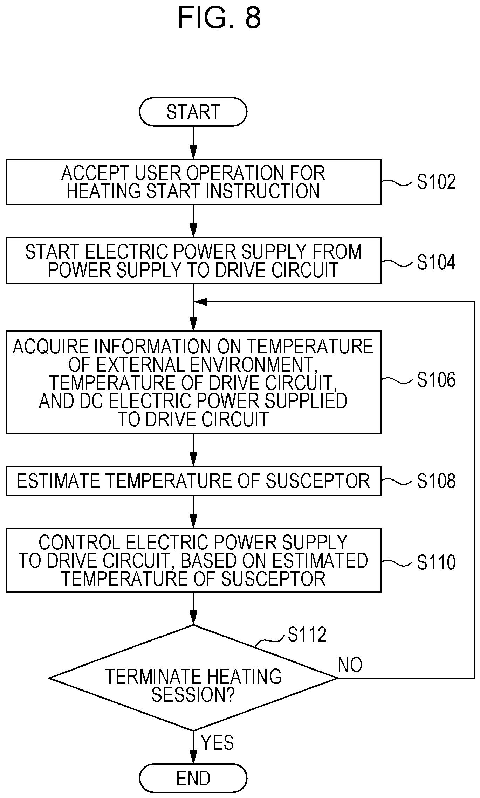

Fig. 8 is a flowchart illustrating an example of a procedure of a process performed by the inhaler device 100 according to the present embodiment.

As illustrated in Fig. 8, first, the sensor 112 accepts a user operation for a heating start instruction (step S102). An example of the operation for instructing the start of heating is pressing of a button of the inhaler device 100. Another example of the operation for instructing the start of heating is insertion of the stick substrate 150 into the inhaler device 100. That is, when the stick substrate 150 is inserted into the inhaler device 100, heating may be automatically started.

Subsequently, the controller 116 starts electric power supply from the power supply 111 to the drive circuit 169 (step S104). At and after this step, the controller 116 controls electric power supply to the electromagnetic induction source 162, based on the heating profile.

Then, the controller 116 acquires the information of the temperature of the external environment, the information of the temperature of the drive circuit 169, and the information of the DC electric power supplied to the drive circuit 169 (step S106). These pieces of information are detected by the first temperature sensor 171, the second temperature sensor 172, and the electric power sensor 173.

Subsequently, the controller 116 estimates the temperature of the susceptor 161, based on the information acquired in step S106 (step S108). Specifically, first, the controller 116 estimates the temperature of the susceptor 161, based on the information of the DC electric power supplied to the drive circuit 169. Subsequently, the controller 116 corrects the estimated temperature of the susceptor 161, based on the temperature of the external environment and the temperature of the drive circuit 169.

Then, the controller 116 controls electric power supply to the drive circuit 169, based on the estimated temperature of the susceptor 161 (step S110). For example, the controller 116 controls electric power supply to the drive circuit 169 such that the estimated temperature of the susceptor 161 changes in the same manner as the time-series change in the target temperature defined in the heating profile.

Subsequently, the controller 116 determines whether to terminate the heating session (step S112). For example, the controller 116 determines to terminate the heating session when the elapsed time from the start of heating (that is, the start of electric power supply to the drive circuit 169) reaches a predetermined value.

If the controller 116 determines not to terminate the heating session (step S112: NO), the process returns to step S106 again. On the other hand, if the controller 116 determines to terminate the heating session (step S112: YES), the process ends.

### <4. Supplementary description>

While the preferred embodiment of the present invention has been described in detail above with reference to the accompanying drawings, the present invention is not limited to such examples. Obviously, a person with an ordinary knowledge in the technical field to which the present invention pertains can conceive various modifications and corrections within the scope of the technical spirit described in the claims. It should be understood that these modifications and corrections naturally pertain to the technical scope of the present invention.

For example, in the embodiment above, an example has been described in which the electrical resistance value Rs of the drive circuit 169 is estimated based on the temperature of the drive circuit 169 detected by the second temperature sensors 172. However, the invention is not limited to such an example. For example, the controller 116 may estimate the electrical resistance value R_{S} of the drive circuit 169, based on the elapsed time from the start of the electric power supply to the drive circuit 169 and information that is stored in advance and indicates a time-series change in the electrical resistance value Rs of the drive circuit 169. The change in the temperature of the drive circuit 169 is caused by electric power supply to the drive circuit 169. When electric power supply to the drive circuit 169 is controlled in accordance with the heating profile, the time-series changes in the temperature and the electrical resistance value Rs of the drive circuit 169 are determined in advance as the time-series changes have a certain relation with the time-series change in the target temperature in the heating profile. Therefore, the controller 116 can estimate the electrical resistance value Rs of the drive circuit 169 corresponding to the current elapsed time from the start of the electric power supply to the drive circuit 169, with reference to the time-series change in the electrical resistance value Rs of the drive circuit 169 determined in advance. With such a configuration, electric power supply control based on the temperature of the drive circuit 169 can be implemented even if the temperature of the drive circuit 169 is not detected by the second temperature sensor 172 and further even if the second temperature sensor 172 itself is not provided.

For example, in the embodiment above, an example has been described in which the susceptor 161 is a piece of metal. However, the present invention is not limited to such an example. For example, the susceptor 161 may have an elongated shape such as a rod shape, a cylindrical shape, or a plate shape. In this case, the susceptor 161 is desirably disposed at the center of the substrate 151 to extend in a longitudinal direction of the substrate 151. With such a configuration, an aerosol can be generated in a short time from the start of heating since the susceptor 161 that produces a large amount of heat by induction heating is disposed at the center of the substrate 151. Obviously, the susceptors 161 having a plurality of shapes may coexist in the substrate 151.

For example, in the embodiment described above, an example has been described in which the substrate 151 includes the susceptor 161. However, the present invention is not limited to such an example. That is, the susceptor 161 may be disposed at any position where the susceptor 161 is in thermal proximity to the aerosol source. In an example, the susceptor 161 may have a blade-like shape, and may be disposed so that the susceptor 161 protrudes from the bottom 143 of the holder 140 toward the internal space 141. When the stick substrate 150 is inserted into the holder 140, the susceptor 161 having the blade-like shape may be inserted so as to pierce the substrate 151 from the end portion of the stick substrate 150 in the insertion direction. In another example, the susceptor 161 may be disposed on an inner wall of the holder 140 that forms the internal space 141.

The series of steps performed by the individual devices described in this specification may be implemented by using any of software, hardware, and a combination of software and hardware. Programs constituting software are, for example, stored in advance in recording media (non-transitory media) provided inside or outside the individual devices. Each program is, for example, at the time of being executed by a computer that controls each of the devices described in this specification, loaded into a RAM and executed by a processor such as a CPU. The recording media are, for example, a magnetic disk, an optical disc, a magneto-optical disk, a flash memory, and the like. The computer programs may be distributed, for example, via a network without using recording media.

The steps described using a flowchart and a sequence diagram in this specification need not necessarily be executed in the order illustrated. Some of the process steps may be executed in parallel. An additional process step may be adopted, or one or some of the process steps may be omitted.

Configurations below also pertain to the technical scope of the present invention.
(1) An inhaler device including:
   a power supply configured to supply DC electric power;
   a drive circuit including an inverter circuit configured to convert the DC electric power supplied from the power supply into AC electric power, and an electromagnetic induction source configured to generate a varying magnetic field by using the AC electric power supplied from the inverter circuit;
   a controller configured to control electric power supply from the power supply to the drive circuit;
   a holder configured to hold a substrate including an aerosol source; and
   an electric power sensor configured to detect information of the DC electric power supplied from the power supply to the drive circuit, in which
   the electromagnetic induction source is disposed at a position where the varying magnetic field generated from the electromagnetic induction source penetrates a susceptor that is disposed in thermal proximity to the aerosol source included in the substrate held by the holder,
   the susceptor is configured to produce heat upon being penetrated by the varying magnetic field, and
   the controller is configured to control the electric power supply to the drive circuit, based on information related to a disturbance and the information of the DC electric power supplied to the drive circuit detected by the electric power sensor.
(2) The inhaler device according to (1), in which
   the controller is configured to estimate a temperature of the susceptor, based on the information related to the disturbance and the information of the DC electric power supplied to the drive circuit, and control the electric power supply to the drive circuit, based on the estimated temperature of the susceptor.
(3) The inhaler device according to (2), in which
   the controller is configured to correct, based on the information related to the disturbance, the temperature of the susceptor estimated based on the information of the DC electric power supplied to the drive circuit.
(4) The inhaler device according to any one of (1) to (3), further including:
   a first temperature sensor configured to detect a temperature of an external environment, in which
   the information related to the disturbance includes the temperature of the external environment.
(5) The inhaler device according to (4), in which
   the controller is configured to correct the temperature of the susceptor estimated based on the information of the DC electric power supplied to the drive circuit, to be lower as the temperature of the external environment is higher and to be higher as the temperature of the external environment is lower.
(6) The inhaler device according to any one (1) to (5), in which
   the information related to the disturbance includes an electrical resistance value of the drive circuit.
(7) The inhaler device according to (6), in which
   the controller is configured to correct the temperature of the susceptor estimated based on the information of the DC electric power supplied to the drive circuit, to be lower as the electrical resistance value of the drive circuit is larger and to be higher as the electrical resistance value of the drive circuit is smaller.
(8) The inhaler device according to (6) or (7), further including:
   a second temperature sensor configured to detect a temperature of the drive circuit, in which
   the controller is configured to estimate the electrical resistance value of the drive circuit, based on the temperature of the drive circuit detected by the second temperature sensor.
(9) The inhaler device according to (6) or (7), in which
   the controller is configured to estimate the electrical resistance value of the drive circuit, based on an elapsed time from a start of the electric power supply to the drive circuit and information that is stored in advance and indicates a time-series change in the electrical resistance value of the drive circuit.
(10) The inhaler device according to any one of (1) to (9), in which
   the controller is configured to control a voltage to be applied to the drive circuit to control the electric power supply to the drive circuit.
(11) The inhaler device according to any one of (1) to (10), in which
   the controller is configured to control, based on a heating profile, the electric power supply to the drive circuit, the heating profile being information that defines a time-series change in a target temperature that is a target value of a temperature of the susceptor.
(12) The inhaler device according to (11), in which
   the controller is configured to perform control to apply a first voltage to the drive circuit in a case where an estimated temperature of the susceptor is lower than the target temperature, and to apply a second voltage to the drive circuit in a case where the estimated temperature of the susceptor is higher than the target temperature, and
   an average value of the first voltage per unit time is higher than an average value of the second voltage per the unit time.
(13) The inhaler device according to (12), in which
   the second voltage is equal to 0.
(14) A program to be executed by a computer that controls an inhaler device,
   the inhaler device including:
   a power supply configured to supply DC electric power;
   a drive circuit including an inverter circuit configured to convert the DC electric power supplied from the power supply into AC electric power, and an electromagnetic induction source configured to generate a varying magnetic field by using the AC electric power supplied from the inverter circuit;
   a holder configured to hold a substrate including an aerosol source; and
   an electric power sensor configured to detect information of the DC electric power supplied from the power supply to the drive circuit,
   the electromagnetic induction source being disposed at a position where the varying magnetic field generated from the electromagnetic induction source penetrates a susceptor that is disposed in thermal proximity to the aerosol source included in the substrate held by the holder,
   the susceptor being configured to produce heat upon being penetrated by the varying magnetic field, the program causing
   controlling electric power supply to the drive circuit, based on information related to a disturbance and the information of the DC electric power supplied to the drive circuit detected by the electric power sensor to be performed.
(15) A system including: an inhaler device; and a substrate,
   the substrate including an aerosol source,
   the inhaler device including:
      a power supply configured to supply DC electric power;
      a drive circuit including an inverter circuit configured to convert the DC electric power supplied from the power supply into AC electric power, and an electromagnetic induction source configured to generate a varying magnetic field by using the AC electric power supplied from the inverter circuit;
      a controller configured to control electric power supply from the power supply to the drive circuit;
      a holder configured to hold the substrate; and
      an electric power sensor configured to detect information of the DC electric power supplied from the power supply to the drive circuit, in which
      the electromagnetic induction source is disposed at a position where the varying magnetic field generated from the electromagnetic induction source penetrates a susceptor that is disposed in thermal proximity to the aerosol source included in the substrate held by the holder,
      the susceptor is configured to produce heat upon being penetrated by the varying magnetic field, and
      the controller is configured to control the electric power supply to the drive circuit, based on information related to a disturbance and the information of the DC electric power supplied to the drive circuit detected by the electric power sensor.
(16) The system according to (15), in which
   the susceptor is included in the substrate.

### Reference Signs List

- 100: inhaler device
- 111: power supply
- 112: sensor
- 113: notifier
- 114: memory
- 115: communicator
- 116: controller
- 140: holder
- 141: internal space
- 142: opening
- 143: bottom
- 150: stick substrate
- 151: substrate
- 152: inhalation port
- 161: susceptor
- 162: electromagnetic induction source
- 163: inverter circuit
- 169: drive circuit
- 171: first temperature sensor
- 172: second temperature sensor
- 173: electric power sensor

## Claims

1. An inhaler device comprising:
a power supply configured to supply DC electric power;
a drive circuit including an inverter circuit configured to convert the DC electric power supplied from the power supply into AC electric power, and an electromagnetic induction source configured to generate a varying magnetic field by using the AC electric power supplied from the inverter circuit;
a controller configured to control electric power supply from the power supply to the drive circuit;
a holder configured to hold a substrate including an aerosol source; and
an electric power sensor configured to detect information of the DC electric power supplied from the power supply to the drive circuit, wherein
the electromagnetic induction source is disposed at a position where the varying magnetic field generated from the electromagnetic induction source penetrates a susceptor that is disposed in thermal proximity to the aerosol source included in the substrate held by the holder,
the susceptor is configured to produce heat upon being penetrated by the varying magnetic field, and
the controller is configured to control the electric power supply to the drive circuit, based on information related to a disturbance and the information of the DC electric power supplied to the drive circuit detected by the electric power sensor.

2. The inhaler device according to claim 1, wherein
the controller is configured to estimate a temperature of the susceptor, based on the information related to the disturbance and the information of the DC electric power supplied to the drive circuit, and control the electric power supply to the drive circuit, based on the estimated temperature of the susceptor.

3. The inhaler device according to claim 2, wherein
the controller is configured to correct, based on the information related to the disturbance, the temperature of the susceptor estimated based on the information of the DC electric power supplied to the drive circuit.

4. The inhaler device according to any one of claims 1 to 3, further comprising:
a first temperature sensor configured to detect a temperature of an external environment, wherein
the information related to the disturbance includes the temperature of the external environment.

5. The inhaler device according to claim 4, wherein
the controller is configured to correct the temperature of the susceptor estimated based on the information of the DC electric power supplied to the drive circuit, to be lower as the temperature of the external environment is higher and to be higher as the temperature of the external environment is lower.

6. The inhaler device according to any one of claims 1 to 5, wherein
the information related to the disturbance includes an electrical resistance value of the drive circuit.

7. The inhaler device according to claim 6, wherein
the controller is configured to correct the temperature of the susceptor estimated based on the information of the DC electric power supplied to the drive circuit, to be lower as the electrical resistance value of the drive circuit is larger and to be higher as the electrical resistance value of the drive circuit is smaller.

8. The inhaler device according to claim 6 or 7, further comprising:
a second temperature sensor configured to detect a temperature of the drive circuit, wherein
the controller is configured to estimate the electrical resistance value of the drive circuit, based on the temperature of the drive circuit detected by the second temperature sensor.

9. The inhaler device according to claim 6 or 7, wherein
the controller is configured to estimate the electrical resistance value of the drive circuit, based on an elapsed time from a start of the electric power supply to the drive circuit and information that is stored in advance and indicates a time-series change in the electrical resistance value of the drive circuit.

10. The inhaler device according to any one of claims 1 to 9, wherein
the controller is configured to control a voltage to be applied to the drive circuit to control the electric power supply to the drive circuit.

11. The inhaler device according to any one of claims 1 to 10, wherein
the controller is configured to control, based on a heating profile, the electric power supply to the drive circuit, the heating profile being information that defines a time-series change in a target temperature that is a target value of a temperature of the susceptor.

12. The inhaler device according to claim 11, wherein
the controller is configured to perform control to apply a first voltage to the drive circuit in a case where an estimated temperature of the susceptor is lower than the target temperature, and to apply a second voltage to the drive circuit in a case where the estimated temperature of the susceptor is higher than the target temperature, and
an average value of the first voltage per unit time is higher than an average value of the second voltage per the unit time.

13. The inhaler device according to claim 12, wherein
the second voltage is equal to 0.

14. A program to be executed by a computer that controls an inhaler device,
the inhaler device including:
a power supply configured to supply DC electric power;
a drive circuit including an inverter circuit configured to convert the DC electric power supplied from the power supply into AC electric power, and an electromagnetic induction source configured to generate a varying magnetic field by using the AC electric power supplied from the inverter circuit;
a holder configured to hold a substrate including an aerosol source; and
an electric power sensor configured to detect information of the DC electric power supplied from the power supply to the drive circuit,
the electromagnetic induction source being disposed at a position where the varying magnetic field generated from the electromagnetic induction source penetrates a susceptor that is disposed in thermal proximity to the aerosol source included in the substrate held by the holder,
the susceptor being configured to produce heat upon being penetrated by the varying magnetic field, the program causing
controlling electric power supply to the drive circuit, based on information related to a disturbance and the information of the DC electric power supplied to the drive circuit detected by the electric power sensor to be performed.

15. A system comprising: an inhaler device; and a substrate,
the substrate including an aerosol source,
the inhaler device including:
a power supply configured to supply DC electric power;
a drive circuit including an inverter circuit configured to convert the DC electric power supplied from the power supply into AC electric power, and an electromagnetic induction source configured to generate a varying magnetic field by using the AC electric power supplied from the inverter circuit;
a controller configured to control electric power supply from the power supply to the drive circuit;
a holder configured to hold the substrate; and
an electric power sensor configured to detect information of the DC electric power supplied from the power supply to the drive circuit, wherein
the electromagnetic induction source is disposed at a position where the varying magnetic field generated from the electromagnetic induction source penetrates a susceptor that is disposed in thermal proximity to the aerosol source included in the substrate held by the holder,
the susceptor is configured to produce heat upon being penetrated by the varying magnetic field, and
the controller is configured to control the electric power supply to the drive circuit, based on information related to a disturbance and the information of the DC electric power supplied to the drive circuit detected by the electric power sensor.

16. The system according to claim 15, wherein
the susceptor is included in the substrate.
